# EUROPEAN PATENT APPLICATION

(11) **EP 1 457 568 A1**
(43) Date of publication of application: **15.09.2004**
(21) Application number: 02786164.0
(22) Date of filing: 20.12.2002
(51) Int. Cl.: C12P 19/40

(54) **PROCESS FOR PRODUCING 2 -DEOXYGUANOSINE**

(30) Priority: 28.12.2001 JP 2001399455; 22.07.2002 JP 2002212348
(71) Applicant: YAMASA CORPORATION, Choshi-shi, Chiba 288-0056 (JP)
(72) Inventor: NOGUCHI, Toshitada, Choshi-shi, Chiba 288-0812 (JP); HAMAMOTO, Tomoki, Choshi-shi, Chiba 288-0033 (JP); OKUYAMA, Kiyoshi, Sanbu-gun, Chiba 289-1345 (JP); SHIBUYA, Susumu, Choshi-shi, Chiba 288-0814 (JP)
(74) Representative: von Kreisler, Alek, Dipl.-Chem.
(86) International application number: PCT/JP2002/013354
(87) International publication number: WO 2003/057895

(57) **Abstract**

The invention provides a process for producing 2'-deoxyguanosine, characterized in that the process includes reacting one compound selected from the group consisting of guanosine, guanosine 5'-monophosphate, and 2-amino-6-substituted purine with 2'-deoxynucleoside in the presence of nucleoside deoxyribosyl transferase and a hydrolase.

According to the process of the present invention, 2'-deoxyguanosine can be synthesized efficiently from inexpensive and easily available starting materials. Since no guanosine, which disturbs purification, is virtually present in a reaction mixture, isolation and purification of 2'-deoxyguanosine can be performed in a very simple manner. Thus, the process for producing 2'-deoxyguanosine is practical.

## Description

### Technical Field

The present invention relates to a process for producing 2'-deoxyguanosine by use of two enzymes; i.e., nucleoside deoxyribosyl transferase and a hydrolase, in combination, or in other words, by use of "coupling" of two enzyme reactions.

### Background Art

2'-Deoxynucleosides are compounds useful for producing a variety of drugs, such as an antisense drug (e.g., 2'-deoxynucleotide oligomer).

Conventionally, 2'-deoxynucleosides have been chemically synthesized or prepared through enzymatic degradation of DNA derived from, for example, milt. Chemical synthesis imposes high production costs, whereas enzymatic degradation of DNA raises the problem that each of the four species of 2'-deoxynucleosides produced has a poor balance between demand and supply, making the method less efficient.

In recent years, several methods have been developed for synthesizing 2'-deoxynucleosides by use of nucleoside phosphorylases. In these enzymatic methods, 2'-deoxyuridine or thymidine, which can be chemically synthesized easily, is employed as a 2-deoxyribose donor in the presence of a chemically synthesizable base of nucleic acid or ribonucleoside as well as phosphate ions, and a target 2'-deoxynucleoside is synthesized through glycosyl transfer reaction by the mediation of the enzymes.

However, among the four species of 2'-deoxynucleoside, 2'-deoxyguanosine is left behind in terms of established effective production method therefor, because of poor solubility of guanine (for example, Japanese Patent Application Laid-Open (*kokai*) No. 11-137290 discloses that the yield of 2'-deoxyguanosine is approximately 1 g/L (3.6 mmol/L)).

Hitherto, several methods have been proposed to solve the above problem:
(1) Method 1: a method for synthesizing 2'-deoxyguanosine through employment of a microorganism as an enzyme (nucleoside phosphorylase) source and, as substrates, 2'-deoxyuridine or thymidine, and guanosine or guanylic acid (Japanese Patent Application Laid-Open (*kokai*) No. 11-137290).
   Although method 1 employs guanosine or guanylic acid as a substrate instead of guanine, 2'-deoxyguanosine is formed in an amount of about 6.3 to 7.3 g/L (22.8 to 26.4 mmol/L) at most. In addition, method 1 also has the problems that a large amount of cultured cells is required and that 2'-deoxyguanosine must be separated for purification from guanosine that is present in the same reaction mixture. However, exclusive separation/purification of 2'-deoxyguanosine from a mixture of guanosine and 2'-deoxyguanosine is virtually impossible, since guanosine and 2'-deoxyguanosine have very similar physical properties. Thus, method 1 cannot be considered a practical method.
(2) Method 2: a method for synthesizing 2'-deoxyguanosine including employing a microorganism as an enzyme (nucleoside phosphorylase) source and, as substrates, 2'-deoxyuridine or thymidine and diaminopurine, to thereby synthesize diaminopurine 2'-deoxyriboside, and treating the 2'-deoxyriboside with adenosine deaminase (Japanese Patent Application Laid-Open (*kokai*) No. 11-137289 and US Patent No. 6,197,552).
   Method 2 employs diaminopurine having higher solubility as a substrate so as to overcome the aforementioned drawback involved in method 1. However, diaminopurine is a relatively expensive compound, and the yield of the target compound is not necessarily satisfactory.
(3) Method 3: a method for synthesizing 2'-deoxyguanosine through reaction of thymidine serving as a 2-deoxyribose donor with guanine in the presence of nucleoside phosphorylase, wherein the formed thymine is enzymatically degraded to thereby shift the reaction equilibrium toward synthesis of 2'-deoxyguanosine (Japanese Patent Application Laid-Open (*kokai*) No. 11-46790 and US Patent No. 6,017,736).
   As has been reported, method 3 can be employed to synthesize 2'-deoxyguanosine at a concentration of about 12 to 15 mmol/L. However, the method requires a peculiar enzyme such as uracil thymine dehydrogenase, and thus is not necessarily considered very practical.
(4) Method 4: a method for synthesizing 2'-deoxyguanosine by employing 2-deoxyribose 1-phosphate and guanine as substrates in the presence of nucleoside phosphorylase serving as a catalyst, wherein calcium chloride is added to the reaction system so as to enhance synthesis efficiency (Japanese Patent Application Laid-Open (*kokai*) No. 2001-26599 and the pamphlet of WO00/70074).
   With regard to method 4, 2-deoxyribose 1-phosphate is difficult to obtain, and reaction yield is as low as about 8 mmol/L. Thus, the method is not suitable for practical use.
(5) Method 5: a method for synthesizing 2'-deoxyguanosine including employing 2-deoxyribose 1-phosphate and glyoxal guanine as substrates in the presence of nucleoside phosphorylase serving as a catalyst, to thereby synthesize glyoxal deoxyguanosine, and decomposing glyoxal deoxyguanosine by an alkali, to thereby form 2'-deoxyguanosine (Japanese Patent Application Laid-Open (*kokai*) No. 2001-269192 and European Patent No. 1,138,775).

In method 5, 2-deoxyribose 1-phosphate is difficult to obtain, and the attainable yield of 2'-deoxyguanosine is not clearly disclosed.

The present inventors previously proposed synthesis of 2'-deoxyguanosine through employment of guanine, and thymidine or 2'-deoxyuridine as substrates in the presence of nucleoside deoxyribosyl transferase serving as a catalyst. However, because of poor solubility of guanine, 2'-deoxyguanosine could be synthesized only at a concentration of about 2 to 3 mmol/L (Japanese Patent Application Laid-Open (*kokai*) No. 2001-46097).

As described above, although a variety of enzymatic synthesis methods for 2'-deoxyguanosine have been studied, a practical synthesis method has not yet been proposed.

Thus, an object of the present invention is to provide an effective and practical process for producing 2'-deoxyguanosine through enzymatic synthesis employing starting materials which are inexpensive and readily available.

### Disclosure of the Invention

The present inventors have carried out extensive studies on a process for producing 2'-deoxyguanosine by use of nucleoside deoxyribosyl transferase, and have found that 2'-deoxyguanosine can be produced efficiently through employment of inexpensive guanosine or guanosine 5'-monophosphate and a 2'-deoxynucleoside other than 2'-deoxyguanosine as substrates, as well as nucleoside deoxyribosyl transferase and nucleosidase, serving as a hydrolase, in combination.

In general, nucleoside deoxyribosyl transferase is considered to recognize guanine as a substrate only when the guanine is in a dissolved state. When guanine, and thymidine or 2'-deoxyuridine are employed as substrates in the presence of nucleoside deoxyribosyl transferase so as to synthesize 2'-deoxyguanosine, the yield can reach only about 2 to 3 mmol/L. Even when guanosine or guanosine 5'-monophosphate (GMP) is decomposed by nucleosidase to form guanine and a ribose (or a ribose 5-phosphate), guanine immediately becomes insoluble and precipitates. Thus, guanine has been conventionally considered unable to serve as a substrate with respect to nucleoside deoxyribosyl transferase.

However, quite surprisingly, the present inventors have confirmed that a process for producing 2'-deoxyguanosine by use of nucleoside deoxyribosyl transferase and a nucleosidase in combination (i.e., coupling of two enzyme reactions) is a practical process which attains high-yield production of 2'-deoxyguanosine and has, among others, the following advantages. <1> Even when insoluble guanosine or guanosine 5'-monophosphate is present, nucleosidase continuously decomposes guanosine or guanosine 5'-monophosphate without causing any problem. <2> Through co-presence of nucleoside deoxyribosyl transferase in the reaction system upon decomposition of guanosine or guanosine 5'-monophosphate in the presence of nucleosidase to thereby form guanine and a ribose or a ribose 5-phosphate, the formed guanine can be immediately recognized as a substrate by nucleoside deoxyribosyl transferase before precipitation of guanine, and 2'-deoxyguanosine is rapidly formed, with virtually no precipitation of guanine being observed. <3> Since nucleosidase acts exclusively on ribonucleoside or ribonucleotide and does not act on 2'-deoxynucleoside, the target 2'-deoxyguanosine is not decomposed by nucleosidase. <4> Since guanosine or guanosine 5'-monophosphate, which disturbs separation and purification of 2'-deoxyguanosine, can be completely decomposed by nucleosidase, 2'-deoxyguanosine is readily separated and purified. The present invention has been accomplished on the basis of these findings.

The present inventors have also found that 2'-deoxyguanosine can be efficiently produced by employing, instead of guanine, 2-amino-6-substituted purine and 2'-deoxynucleoside, as well as nucleoside deoxyribosyl transferase and a hydrolase in combination.

As mentioned above, synthesis of deoxynucleoside compounds by use of nucleoside phosphorylase and adenosine deaminase in combination has conventionally been reported in a variety of documents (for example, Japanese Patent Application Laid-Open (*kokai*) No. 11-137289, US Patent 6,197,552, and Japanese Patent No. 3,033,918). However, production of a deoxynucleoside compound by use of nucleoside deoxyribosyl transferase and a hydrolase in combination has not been reported.

The present inventors have found that use of nucleoside deoxyribosyl transferase attains higher reaction efficiency as compared with nucleoside phosphorylase, and have confirmed that 2'-deoxyguanosine can be efficiently produced by use of nucleoside deoxyribosyl transferase and a hydrolase in combination. The present invention has been also accomplished on the basis of this finding.

Accordingly, the present invention provides a process for producing 2'-deoxyguanosine, which comprises reacting one compound selected from the group consisting of guanosine, guanosine 5'-monophosphate, and 2-amino-6-substituted purine with 2'-deoxynucleoside in the presence of nucleoside deoxyribosyl transferase and a hydrolase.

### Best Modes for Carrying Out the Invention

No particular limitation is imposed on the nucleoside deoxyribosyl transferase employed in the present invention, and any nucleoside deoxyribosyl transferase can be employed so long as the transferase has a 2-deoxyribosyl group transfer activity. Examples include nucleoside deoxyribosyl transferase I and nucleoside deoxyribosyl transferase II.

The hydrolase employed in the present invention is a nucleosidase or an enzyme for hydrolyzing a 6-substituent of 2-amino-6-substituted purine (hereinafter the enzyme may be referred to as 6-substituent hydrolase).

The process of the present invention for producing 2'-deoxyguanosine includes the following three modes, in accordance with the types of the hydrolase and compounds employed.

In the case where a nucleosidase is employed as the hydrolase, either of the following two processes may be employed.

A first process for producing 2'-deoxyguanosine includes reacting guanosine and 2'-deoxynucleoside in the presence of nucleosidase and nucleoside deoxyribosyl transferase.

Specifically, guanosine (R-Gua) is hydrolyzed with nucleosidase (E1), to thereby form guanine, and directly thereafter or simultaneously, guanine is reacted with 2'-deoxynucleoside (dR-B) in the presence of nucleoside deoxyribosyl transferase (E2), to thereby produce 2'-deoxyguanosine (dR-Gua).

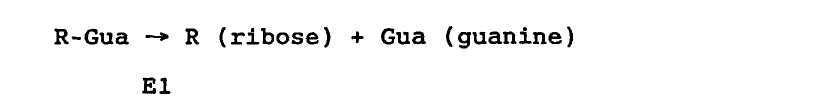

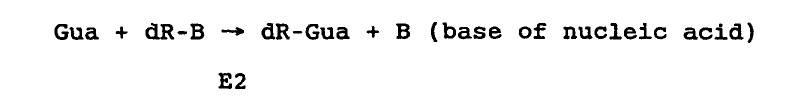

A second process for producing 2'-deoxyguanosine includes reacting guanosine 5'-monophosphate and 2'-deoxynucleoside in the presence of nucleosidase and nucleoside deoxyribosyl transferase.

Specifcally, guanosine 5'-monophosphate (P-R-Gua) is hydrolyzed with nucleosidase (E1), to thereby form guanine, and directly thereafter or simultaneously, guanine is reacted with 2'-deoxynucleoside (dR-B) in the presence of nucleoside deoxyribosyl transferase (E2), to thereby produce 2'-deoxyguanosine (dR-Gua).

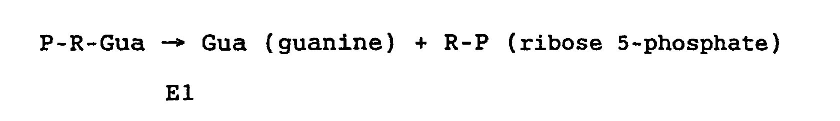

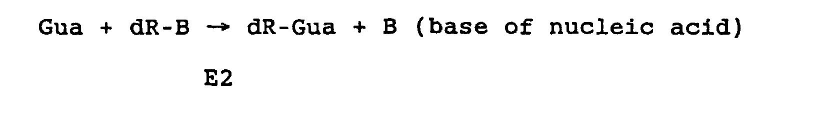

Any enzyme can be employed as the nucleosidase so long as the enzyme has an activity of hydrolyzing nucleoside or nucleotide into a base of nucleic acid and a sugar or a sugar phosphate. Examples include purine nucleosidase and inosinate nucleosidase.

A third process is employed in the case where an enzyme for hydrolyzing a 6-substituent of 2-amino-6-substituted purine is employed as the hydrolase. The 6-substituent hydrolase also includes an enzyme for hydrolyzing a 6-substituent of a nucleoside containing 2-amino-6-substituted purine. The third process for producing 2'-deoxyguanosine includes reacting 2-amino-6-substituted purine and 2'-deoxynucleoside in the presence of nucleoside deoxyribosyl transferase and a 6-substituent hydrolase.

Specifically, 2-amino-6-substituted purine (2-AP) is reacted with 2'-deoxynucleoside (dR-B) in the presence of nucleoside deoxyribosyl transferase (E2), to thereby form 2-amino-6-substituted purine-2'-deoxyriboside (dR-2-AP), and subsequently, the 6-substituent of the product is hydrolyzed by a 6-substituent hydrolase (E3), to thereby produce 2'-deoxyguanosine (dR-Gua).

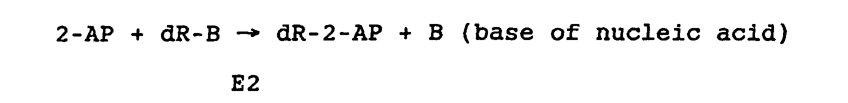

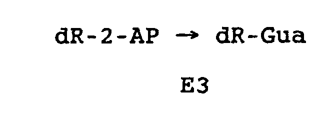

No particular limitation is imposed on the 6-substituent hydrolase, and any enzyme can be employed so long as the enzyme has an activity of hydrolyzing a 6-substituent of 2-amino-6-substituted purine or of a nucleoside containing the purine, to thereby form 2-amino-6-oxopurine (guanine) or a nucleoside containing guanine. Specific examples include deaminase, more specifically adenosine deaminase.

Preferred combinations of nucleoside deoxyribosyl transferase and a hydrolase employed in the present invention are as follows: a combination of nucleoside deoxyribosyl transferase II and a nucleosidase acting on purine nucleoside (purine nucleosidase) (first production process); a combination of nucleoside deoxyribosyl transferase II and inosinate nucleosidase (second production process); and a combination of nucleoside deoxyribosyl transferase II and adenosine deaminase (third production process).

Known enzymes may be used in the present invention, and no particular limitation is imposed on the enzymes. Not only specific enzymes originating from animals, plants, or microorganism, those originating from any origin may be employed. From the viewpoint of facility in enzyme preparation, an enzyme originating from a microorganism is preferably employed. Specifically, nucleoside deoxyribosyl transferase can be readily prepared from a microorganism belonging to lactic acid bacteria, and nucleosidase and adenosine deaminase can be readily prepared from a microorganism belonging to bacteria, yeasts, or molds (see, for example, Methods in Enzymology, Vol. LI. 446 (1978), J. Am. Chem. Soc., 79, 630-633 (1957), J. Biol. Chem., 276, 884-894 (2001), Bull. Agric. Chem. Soc. Japan, 23, 281-288 (1959), and J. Biol. Chem., 242, 740-746 (1967)).

The culture medium employed in the present invention for culturing a microorganism contains appropriate amounts of a carbon source and a nitrogen source which the microorganism can assimilate as well as other ingredients such as a metal salt, a trace growth promoter, and a defoaming agent, in accordance with needs. Specific examples of culture medium ingredients include sugars (glucose, saccharose, etc.), naturally occurring carbohydrates (refinery molasses, crude molasses, starch, wheat, bran, rice, etc.), alcohols, fatty acids, and hydrocarbons. Examples of the nitrogen source include meat extract, yeast extract, and soy bean hydrolyzate. Examples of the metal salt include phosphates, hydrochlorides, and sulfates of zinc, iron, magnesium, or similar metal. Examples of the trace growth promoter include vitamin B1, vitamin B2, and biotin.

Culturing is performed at 20 to 50°C through a routine liquid culturing method (shake culture, aerobic mixing culture, stationary culture, continuous culture, etc.) or a solid culturing method with optionally aerating and stirring in accordance with needs, until the target enzyme activity is fully attained.

The thus-produced culture is appropriately treated in accordance with the purpose of use, thereby yielding an enzyme preparation employed in the present invention. No particular limitation is imposed on the enzyme preparation, and examples include a culture of microorganisms; cells separated from a culture through a routine separation method (centrifugation, precipitation, agglutination, washing, water extraction, etc.); treated products of such cells; and enzyme extracts.

More specifically, the treated products of cells may be obtained by suspending viable cells in an appropriate buffer and physically disrupting the cells through ultrasonication or by means of a French press. Alternatively, the treated products may be obtained by enzymatically (e.g., by lysozyme) causing lysis of the cells and removing cell debris by centrifugation, thereby preparing a cell-free extract. A purified enzyme and a finely purified enzyme which is obtained by subjecting the cell-free extract or the aforementioned enzyme extract to one or more treatments generally employed for purifying enzymes (e.g., heating, salting out by use of ammonium sulfate, dialysis, solvent (e.g., ethanol) treatment, and chromatographic treatments) may also be employed in the present invention.

Alternatively, in the case where a cloned gene of the aforementioned enzyme is available, the target enzyme can be prepared through known recombinant DNA techniques by use of the cloned DNA fragments (see, for example, Science, 277, (5331), 1453-1474, (1997)). Cloning of a gene, preparation of an expression vector by use of cloned DNA fragments, preparation of an enzyme having a target enzyme activity by use of the expression vector, and similar operations are techniques known to those skilled in the molecular biology, and these operation can be carried out through, for example, a method described in "Molecular Cloning" (edited by Maniatis *et al.*, Cold Spring Harbor Laboratories, Cold Spring Harbor, New York (1982)).

Notably, cloning and expression of a gene for nucleoside deoxyribosyl transferase originating from lactic acid bacteria are described in Biosci. Biotechnol. Biochem., 64, 2234-2245 (2000), and cloning and expression of a gene for nucleosidase originating from *E. coli* are described in J. Biol. Chem., 276, 884-894 (2001). Cloning and expression of a gene for adenosine deaminase originating from *E. coli* are described in Japanese Patent Application Laid-Open (*kokai*) No. 5-219978.

Guanosine and guanosine 5'-monophosphate employed in the first and second processes may be commercial products.

As the 2-amino-6-substituted purine employed in the third process, any 2-amino-6-substituted purine derivative may be employed so long as the 6-substituent is hydrolyzable. Specific examples include 2-amino-6-halogenopurine and 2,6-diaminopurine. Among 2-amino-6-halogenopurines, 2-amino-6-chloropurine is preferred.

As the 2'-deoxynucleoside employed as a 2-deoxyribose donor in the present invention, 2'-deoxyadenosine, 2'-deoxycytidine, 2'-deoxyuridine, thymidine, or a similar commercial product may be employed. Of these, 2'-deoxypyrimidinenucleoside is preferred, with thymidine being particularly preferred.

### Synthesis of 2'-deoxyguanosine:

According to the first or second production process, 2'-deoxyguanosine is preferably synthesized in the following procedure. Guanosine or guanosine 5'-monophosphate and 2'-deoxynucleoside are dissolved or suspended in water or a buffer (pH: 3 to 10) so as to adjust the concentration of each ingredient to 10 mmol/L or more, preferably 20 mmol/L or more. In the presence of a nucleosidase and nucleoside deoxyribosyl transferase at a concentration of 0.001 units/mL or more each, preferably 0.01 units/mL or more each, the mixture is allowed to react at 10°C to 70°C, preferably 30°C to 65°C for about 10 minutes to about 50 hours, with optionally stirring the mixture.

In the reaction, co-presence of nucleoside deoxyribosyl transferase in the reaction system is essential during decomposition of guanosine or guanosine 5'-monophosphate in the presence of a nucleosidase into guanine and ribose or ribose 5-phosphate. Before the formed guanine begins precipitating, the guanine is recognized as a substrate by nucleoside deoxyribosyl transferase. Thus, 2'-deoxyguanosine is rapidly formed, and precipitation of guanine does not virtually occur.

When conditions for synthesizing 2'-deoxyguanosine are controlled such that (1) the enzyme activity of nucleoside deoxyribosyl transferase is at least twice higher, preferably at least five times higher than that of the nucleosidase and that (2) the concentration of 2'-deoxynucleoside is at least higher, preferably at least twice higher than that of guanosine or guanosine 5'-monophosphate, 2'-deoxyguanosine can be synthesized at high yield. These optimum conditions can be determined by a small-scale experiment.

According to the third production process, the 2'-deoxyguanosine synthesis reaction includes two reaction steps; i.e., reaction caused by nucleoside deoxyribosyl transferase and reaction caused by a hydrolase. These two reaction steps may be performed simultaneously in parallel or sequentially.

Specifically, 2'-deoxynucleoside and a 2-amino-6-substituted purine are dissolved or suspended in water or a buffer so as to have a concentration of each ingredient of 10 mmol/L or more, preferably 20 mmol/L or more. 2-Amino-6-substituted purine-2'-deoxyriboside serving as an intermediate is prepared by use of nucleoside deoxyribosyl transferase. The intermediate, which may be isolated or may be used as is, is treated with a hydrolase such as adenosine deaminase in an aqueous medium, thereby producing the target 2'-deoxyguanosine.

In the above reactions, nucleoside deoxyribosyl transferase is employed in an amount of 0.001 units/mL or more, preferably 0.01 units /mL or more, and adenosine deaminase is employed in an amount of 2 units/mL or more, preferably 20 units/mL or more. The reactions are performed preferably under the following conditions: pH of 3 to 10, preferably 5 to 9, temperature of 10 to 60°C, preferably 20 to 50°C, for about 10 minutes to about 50 hours, with optionally stirring in accordance with needs.

In the aforementioned reactions, when 2-amino-6-substituted purine-2'-deoxyriboside is not isolated from the reaction mixture and then treated with a hydrolase, the nucleoside deoxyribosyl transferase is preferably inactivated through inactivation treatment such as heating or alkali treatment prior to treatment with a hydrolase.

In each of the above reactions, when the reaction temperature is lower than 10°C, slow reaction rate impairs reaction efficiency, whereas when the reaction temperature is higher than 70°C, enzymes may be denatured or inactivated. Needless to say, both cases are not preferred. The pH of the reaction system varies in the course of reaction. In this case, the variation is compensated by use of acid or base so that the pH falls within the aforementioned range.

### Purification of 2'-deoxyguanosine:

The thus-formed 2'-deoxyguanosine may be isolated and purified through a method generally employed as a purification method for nucleic acid-related substances or through a modified method thereof. Examples include chromatographic methods such as ion exchange chromatography, adsorption chromatography, partition chromatography, and gel chromatography; methods based on liquid-liquid partition such as countercurrent distribution or countercurrent extraction; condensation; cooling; and methods employing difference in solubility added organic solvent. These isolation/purification methods, which are generally employed in isolation and purification of 2'-deoxynucleoside, may be used singly or in suitable combination.

### Examples

The present invention will next be described in detail by way of examples, which should not be construed as limiting the invention thereto.

In the examples, preparation of DNA, cleavage by use of a restriction enzyme, ligation of DNA by use of T4DNA ligase, and transformation of *E. coli* were performed in accordance with the aforementioned "Molecular Cloning." Restriction enzymes, T4DNA ligase, and other items were obtained from Takara Shuzo Co., Ltd.

Determination of enzyme activity and calculation of unit of enzyme activity were carried out through the following procedure.

### <Determination of nucleoside deoxyribosyl transferase activity>

An enzyme sample was added to a 20 mmol/L MOPS-sodium hydroxide buffer (pH 6.0) containing thymidine (5 mmol/L) and cytosine (5 mmol/L), and the mixture was maintained at 40°C. After completion of reaction, the enzyme was inactivated through boiling for one minute. The amount of 2'-deoxycytidine that has been formed was quantitated by HPLC. The activity of the enzyme that forms 1 µmole of 2'-deoxycytidine at 40°C in one minute was defined as an activity of 1 unit.

### <Determination of nucleosidase activity>

An enzyme sample was added to a 20 mmol/L MOPS-sodium hydroxide buffer (pH 6.5) containing guanosine(5 mmol/L) or guanosine 5'-monophosphate (5 mmol/L), and the mixture was maintained at 37°C. Reaction was terminated by addition of an equivolume of 0.1 mol/L sodium hydroxide. The amount of guanine formed in the reaction mixture was quantitated by HPLC. The activity of the enzyme that forms 1 µmole of guanine at 37°C in one minute was defined as an activity of 1 unit.

### <Determination of adenosine deaminase activity>

A reaction mixture (5 mL) containing 30 mmol/L adenosine (50 mmol/L Tris-HCl (pH 8.2)) was heated at 37°C in advance, and a cell-free extract (5 µL) was added thereto. The mixture was allowed to react at 37°C for five minutes. An aliquot (0.2 mL) was sampled from the reaction mixture and mixed with an equivolume of 0.1 mmol/L sodium hydroxide so as to inactivate the enzyme. The resultant solution was 166 times diluted, and the amount of inosine formed was determined by HPLC. The activity of the enzyme that forms 1 µmole of inosine at 37°C in one minute was defined as an activity of 1 unit.

### <Determination of purine nucleoside phosphorylase activity and pyrimidine nucleoside phosphorylase activity>

In accordance with the method described in Japanese Patent Application Laid-Open (*kokai*) No. 4-4882, phosphorylase activity with respect to inosine and that with respect to uridine were determined at 70°C and 50°C, respectively.

### Example 1

### (1) Preparation of nucleoside deoxyribosyl transferase II

In accordance with a method described in Biosci. Biotechnol. Biochem., 64, 2243-2245 (2000), cloning of a gene for nucleoside deoxyribosyl transferase (ndtB) was performed by use of *Lactobacillus helveticus* ATCC 8018 strains, thereby producing expression plasmid pTrc-T2F4.

*E. coli* (JM109) carrying plasmid pTrc-T2F4 was inoculated to a 2xTY medium, and incubated at 37°C for two hours. Subsequently, IPTG was added to the culture broth such that the final concentration was adjusted to 0.1 mmol/L, followed by culturing for five hours and for 16 hours at 25°C, whereby the ndtB gene was expressed in a large amount. The cells were collected by centrifugation and suspended in a solution (10 mmol/L Tris-HCl (pH 8.0) and 1 mmol/L EDTA) for disrupting. The suspended cells were disrupted with ultrasonication, followed by centrifugation, to thereby prepare a cell-free extract (specific activity: 36.18 units/mg-protein).

In accordance with a method described in the aforementioned literature, the thus-prepared extract was partially purified through ammonium sulfate fractionation and ion exchange chromatography, to thereby prepare an enzyme sample (specific activity: 36.69 units/mg-protein).

### (2) Preparation of E. coli nucleosidase

### (2-1) Cloning of E. coli nucleosidase yaaF gene

Chromosomal DNA of *E. coli* JM105 strains (ATCC 47016) was prepared in accordance with Saito-Miura's method (Biochim. Biophys. Acta., 72, 619 (1963)). Through employment of the thus-prepared DNA as a template, the following two primer DNAs were synthesized in a routine manner. *E. coli* nucleosidase yaaF gene was amplified through PCR (J. Biol. Chem., 276, 884-894 (2001)).

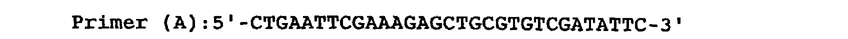

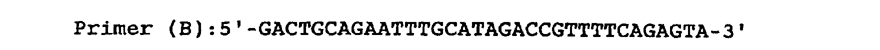

The PCR amplification of the yaaF gene was performed by use of a reaction mixture (50 mmol/L potassium chloride, 10 mmol/L Tris-HCl (pH 8.3), 1.5 mmol/L magnesium chloride, 0.001% gelatin, template DNA 0.1 mg, primer DNA (A) or (B) 0.2 mmol/L, and AmpliTaq DNA polymerase 2.5 units) (in 100 µL) and a DNA Thermal Cycler (product of Perkin-ELmer Cetus Instrument). The PCR process included the steps of thermal denaturation (94°C, 1 min), annealing (57°C, 1.5 min), and polymerization (72°C, 3 min), and the set of the steps was repeated 25 times.

After completion of gene amplification, the reaction mixture was treated with a mixture of phenol/chloroform (1 : 1), and ethanol was added to the obtained aqueous fraction (ethanol : aqueous fraction = 2 : 1 (vol.)), to thereby precipitate DNA. The precipitated DNA was collected and subjected to agarose gel electrophoresis in accordance with the literature (the aforementioned Molecular Cloning), whereby DNA fragments corresponding to 1.2 kb were purified. The DNA was cleaved by use of restriction enzymes EcoRI and PstI. Plasmid pTrc99A (product of Pharmacia Biotech.) digested with the same restriction enzymes EcoRI and PstI was ligated to the cleaved DNA by use of T4DNA ligase. *E. coli* JM109 strains were transformed with the ligation mixture, and plasmid pTrc-yaaF was isolated from the obtained ampicillin-resistant transformant.

The plasmid pTrc-yaaF contains an EcoRI-PstI DNA fragment having *E. coli* yaaF structural gene at the EcoRI-PstI sites that are located downstream of the trc promoter of pTrc99A.

### (2-2) Preparation of E. coli nucleosidase (YaaF)

*E. coli* (JM109) carrying plasmid pTrc-yaaF was inoculated to a 2xTY medium (100 mL) containing 100 µL/mg of ampicillin, and shaking cultivation was performed at 37°C. When the number of cells reached 4 × 10⁸ cells/mL, IPTG was added to the culture broth such that the final concentration was adjusted to 0.2 mmol/L, followed by shaking cultivation for eight hours at 37°C.

After completion of culturing, the cells were collected by centrifugation (9,000×g, 10 min) and suspended in a buffer (10 mL) (20 mmol/L sodium acetate (pH 6.0) and 1 mmol/L magnesium chloride). The cells were disrupted with ultrasonication, followed by centrifugation (20,000×g, 10 min), to thereby remove cell debris. The thus-obtained cell-free extract was dialyzed twice against a solution (1 L) of 20 mmol/L sodium acetate (pH 6.0) containing 1 mmol/L magnesium chloride. The dialyzate collected through a dialysis tube was centrifuged (20,000×g, 10 min), to thereby remove precipitates. The thus-obtained supernatant fraction was adsorbed to a resin column (50 mL) (DEAE-Toyopearl 650S, product of Toso), and a portion of the sample which had not been adsorbed was eluted by the same buffer. Subsequently, the adsorbed sample was eluted with the same buffer containing 200 mmol/L sodium chloride with a linear gradation of salt.

Fractions exhibiting nucleosidase activity were collected and combined, and the combined fraction was dialyzed against the same buffer (1 L). The dialyzed solution was adsorbed to a MonoQ column (1 mL; product of Amersham-Pharmacia). A portion of the sample which had not been adsorbed was eluted by the same buffer, and subsequently, the adsorbed sampled was eluted with the same buffer containing 200 mmol/L sodium chloride. The fractions exhibiting nucleosidase activity were pooled and dialyzed against the same buffer (1 L), and then employed as an enzyme sample. The enzyme sample was found to have a nucleosidase activity of 5.2 units/mg-protein.

### (3) Synthesis of 2'-deoxyguanosine by use of thymidine

To 20 mmol/L acetate buffer (pH 6.0) containing 70 mmol/L thymidine and 35 mmol/L guanosine, enzyme samples were added so as to attain the nucleoside deoxyribosyl transferase II activity of 0.30 units/mL and the nucleosidase activity of 0.06 units/mL. The mixture was incubated at 42°C for 32 hours with stirring. After reaction, formation of 31.0 mmol/L of 2'-deoxyguanosine was confirmed, and no guanosine was detected.

### Example 2

### (1) Preparation of inosinate nucleosidase

Inosinate nucleosidase is prepared from *Aspergillus oryzae* in accordance with a method described in a document (Bull. Agric. Chem. Soc. Japan, 23, 281-288 (1959)).

### (2) Synthesis of 2'-deoxyguanosine by use of thymidine

To 20mM MOPS-NaOH buffer (pH 6.5) containing 25 mmol/L thymidine and 25 mmol/L guanosine 5'-monophosphate, enzyme samples are added so as to attain the nucleoside deoxyribosyl transferase II activity of 0.33 units/mL and the inosinate nucleosidase activity of 0.15 units/mL. The mixture is incubated at 37°C for about 18 hours with stirring, to thereby produce 2'-deoxyguanosine.

### Example 3

### (1) Preparation of nucleoside deoxyribosyl transferase II

To a nutrient medium (100 mL) containing peptone (20 g/L), yeast extract (10 g/L), sodium chloride (5 g/L), glucose (1 g/L), and ampicillin (100 µg/mL), *E. coli* transformant (JM109[pTrc-T2F4]) carrying a recombinant vector pTrc-T2F4 which had been prepared by a method described in Japanese Patent Application Laid-Open (*kokai*) No. 2002-17393 was inoculated, and shaking cultivation was performed at 37°C.

When the number of cells reached 4 × 10⁸ cells/mL, IPTG was added to the culture broth such that the final concentration was adjusted to 0.1 mmol/L, and shaking cultivation was continued for five hours at 37°C. After completion of culturing, cultured cells were collected by centrifugation (9,000×g, 10 min), and the cells were suspended in distilled water (10 mL). The cell suspension was treated with an ultrasonic crusher, and cell debris were removed by centrifugation (12,000×g, 10 min). The thus-obtained supernatant fraction was employed as an enzyme solution.

### (2) Preparation of purine nucleoside phosphorylase

To a nutrient medium (100 mL) containing peptone (20 g/L), yeast extract (10 g/L), sodium chloride (5 g/L), glucose (1 g/L), and ampicillin (100 µg/mL), *E. coli* (JM109[pTrc-B56]) carrying a recombinant vector pTrc-B56 which had been prepared by a method described in Japanese Patent Application Laid-Open (*kokai*) No. 9-117298 was inoculated, and shaking cultivation was performed at 37°C.

When the number of cells reached 4 × 10⁸ cells/mL, IPTG was added to the culture broth such that the final concentration was adjusted to 1 mmol/L, and shaking cultivation was continued for five hours at 37°C. After completion of culturing, cultured cells were collected by centrifugation (9,000×g, 10 min), and the cells were suspended in a buffer (20 mL) (50 mmol/L Tris-HCl buffer (pH 7.8) containing 5 mmol/L EDTA and 0.1% Triton X100). Lysozyme was added to the suspension such that the final concentration was adjusted to 1 mg/mL, and the mixture was maintained at 37°C for one hour, to thereby cause lysis of the transformant. Cell debris were removed by centrifugation (12,000×g, 10 min). The thus-obtained supernatant fraction was employed as an enzyme solution.

### (3) Preparation of pyrimidine nucleoside phosphorylase

To a nutrient medium (100 mL) containing peptone (20 g/L), yeast extract (10 g/L), sodium chloride (5 g/L), glucose (1 g/L), and ampicillin (100 µg/mL), *E. coli* (JM109[pTrc-pyn]) carrying a recombinant vector pTrc-pyn which had been prepared by a method described in Japanese Patent Application Laid-Open (*kokai*) No. 6-253854 was inoculated, and shaking cultivation was performed at 37°C.

When the number of cells reached 4 × 10⁸ cells/mL, IPTG was added to the culture broth such that the final concentration was adjusted to 0.1 mmol/L, and shaking cultivation was continued for 16 hours at 37°C. After completion of culturing, cultured cells were collected by centrifugation (9,000×g, 10 min), and the cells were suspended in a buffer (20 mL) (50 mmol/L Tris-HCl buffer (pH 7.8) containing 5 mmol/L EDTA and 0.1% Triton X100). Lysozyme was added to the suspension such that the final concentration was adjusted to 1 mg/mL, and the mixture was maintained at 37°C for one hour, to thereby cause lysis of the transformant. Cell debris were removed by centrifugation (12,000×g, 10 min). The thus-obtained supernatant fraction was employed as an enzyme solution.

### (4) Preparation of adenosine deaminase

To a nutrient medium (100 mL) containing peptone (20 g/L), yeast extract (10 g/L), sodium chloride (5 g/L), glucose (1 g/L), and ampicillin (100 µg/mL), *E. coli* transformant (JM105[pDR-add]) carrying a recombinant vector pDR-add which had been prepared by a method described in Japanese Patent Application Laid-Open (*kokai*) No. 5-219978 was inoculated, and shaking cultivation was performed at 37°C.

When the number of cells reached 4 × 10⁸ cells/mL, IPTG was added to the culture broth such that the final concentration was adjusted to 0.1 mmol/L, and shaking cultivation was continued for three hours at 37°C. After completion of culturing, cultured cells were collected by centrifugation (9,000×g, 10 min), and the cells were suspended in a buffer (10 mL) (20 mmol/L Tris-HCl buffer (pH 8.2) and 10% ethylene glycol). The cell suspension was treated with an ultrasonic crusher, and cell debris were removed by centrifugation (2,000×g, 10 min). The thus-obtained supernatant fraction was employed as an enzyme solution.

### (5) Synthesis of 2-amino-6-chloropurine-2'-deoxyriboside (Comparison of mole yields in terms of enzymes employed)

A solution (5 mL) containing thymidine (100 mmol/L) (product of YAMASA CORPORATION), 2-amino-6-chloropurine (50 mmol/L) (product of Sigma), and nucleoside deoxyribosyl transferase II (0.385 units/mL) or a combination of purine nucleoside phosphorylase II (0.77 units/mL) and pyrimidine nucleoside phosphorylase (0.385 units/mL) was incubated at 40°C or 50°C for 16 hours with stirring. The results are shown in Table 1.

As is clear from Table 1, 2-amino-6-chloropurine-2'-deoxyriboside can be synthesized with higher yield by use of nucleoside deoxyribosyl transferase, as compared with use of nucleoside phosphorylase. As used herein, mole yield represents the percentage of the amount by mol of 2-amino-6-chloropurine consumed in the synthesis of 2-amino-6-chloropurine-2'-deoxyriboside.

**Table 1**

| Enzyme | 2-amino-6-chloropurine-2'-deoxyriboside | Mole yield (%) |
|---|---|---|
| Nucleoside deoxyribosyl transferase II | 46.4 mmol/L | 92.8 |
| Purine nucleoside phosphorylase II and pyrimidine nucleoside phosphorylase | 39.1 mmol/L | 78.1 |

### (6) Synthesis of 2'-deoxyguanosine

To the reaction mixture (1 mL) obtained by use of nucleoside deoxyribosyl transferase II mentioned in (5) above, 20% sodium hydroxide (10 µL), 1 mol/L Tris-HCl buffer (pH 7.0) (0.1 mL), and adenosine deaminase (50 units/mL) were sequentially added, and the mixture was incubated at 40°C for 13 hours with stirring.

As a result, 2'-deoxyguanosine (42.0 mmol/L) was synthesized from 2-amino-6-chloropurine-2'-deoxyriboside (43.0 mmol/L) (mole yield: 97.2%).

### Example 4

### (1) Synthesis of 2,6-diaminopurine-2'-deoxyriboside

A solution (5 mL) containing thymidine (100 mmol/L) (product of YAMASA CORPORATION), 2,6-diaminopurine (50 mmol/L) (product of sigma), and nucleoside deoxyribosyl transferase II (0.385 units/mL) was incubated at 40°C for 16 hours with stirring. As a result, 2,6-diaminopurine-2'-deoxyriboside was synthesized in an amount of 48.2 mmol/L.

### (2) Synthesis of 2'-deoxyguanosine

To the reaction mixture (1 mL) obtained in (1) above, 20% sodium hydroxide (10 µL), 1 mol/L Tris-HCl buffer (pH 7.0) (0.1 mL), and adenosine deaminase (50 units/mL) were sequentially added, and the mixture was incubated at 40°C for 13 hours with stirring.

As a result, 2'-deoxyguanosine (41.2 mmol/L) was synthesized from 2,6-diaminopurine-2'-deoxyriboside (43.9 mmol/L) (mole yield: 93.8%).

### Industrial Applicability

According to the process of the present invention employing nucleoside deoxyribosyl transferase and a hydrolase in combination (coupled), 2'-deoxyguanosine can be synthesized efficiently from inexpensive and easily available starting materials. Since no guanosine, which disturbs purification, is virtually present in a reaction mixture, isolation and purification of 2'-deoxyguanosine can be performed in a very simple manner. Thus, the process for producing 2'-deoxyguanosine is practical.

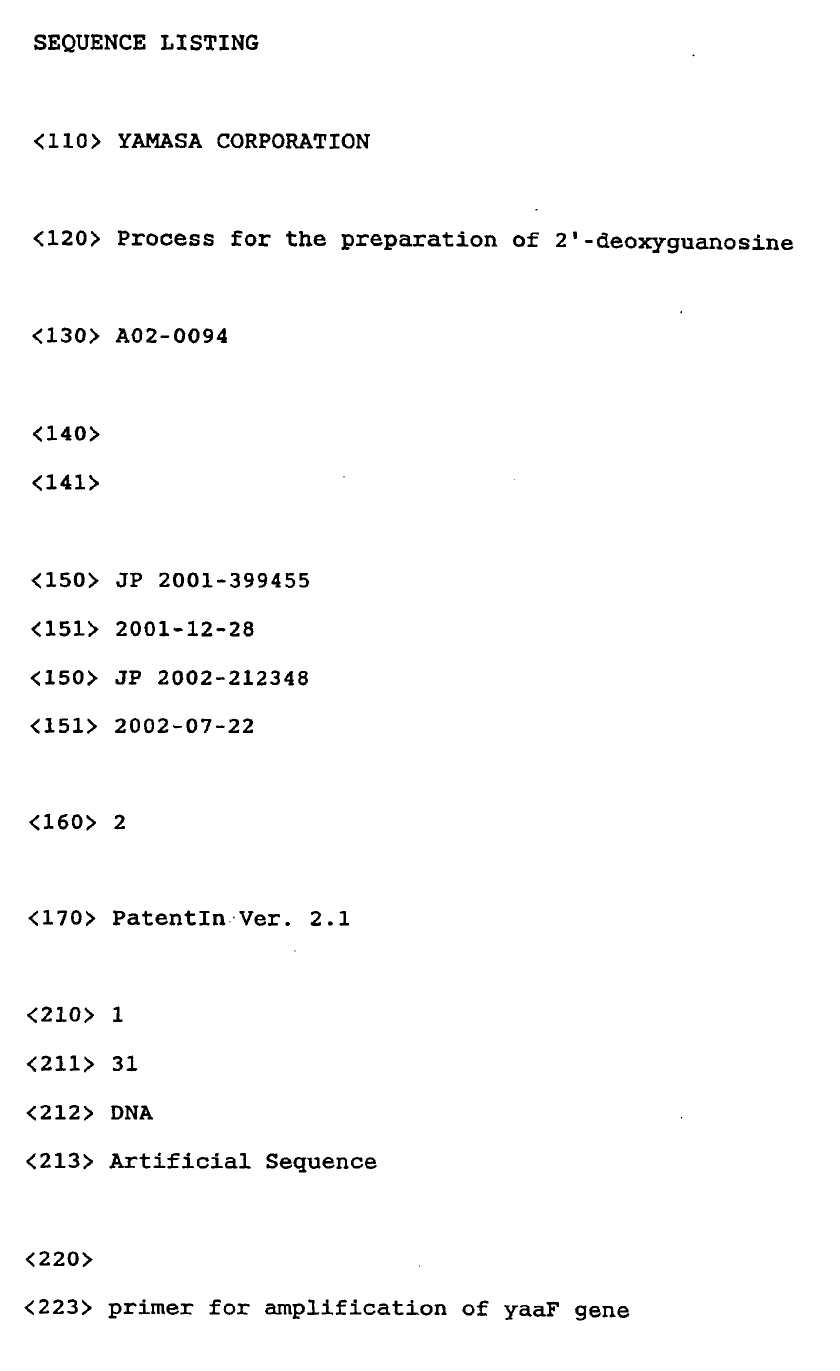

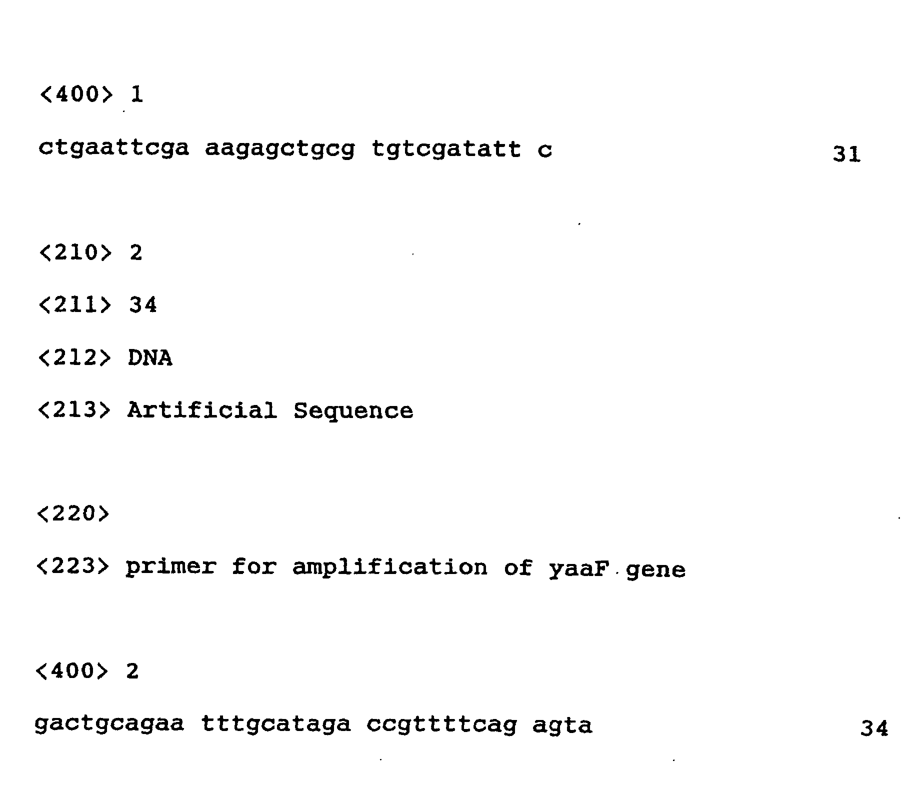

## Claims

1. A process for producing 2'-deoxyguanosine, which comprises reacting one compound selected from the group consisting of guanosine, guanosine 5'-monophosphate, and 2-amino-6-substituted purine with 2'-deoxynucleoside in the presence of nucleoside deoxyribosyl transferase and a hydrolase.

2. A process for producing 2'-deoxyguanosine as described in claim 1, wherein the compound is guanosine and the hydrolase is a nucleosidase.

3. A process for producing 2'-deoxyguanosine as described in claim 2, wherein the nucleoside deoxyribosyl transferase is nucleoside deoxyribosyl transferase II; the nucleosidase is purine nucleosidase; and the 2'-deoxynucleoside is 2'-deoxypyrimidine nucleoside.

4. A process for producing 2'-deoxyguanosine as described in claim 2 or 3, wherein the 2'-deoxynucleoside is thymidine.

5. A process for producing 2'-deoxyguanosine as described in claim 1, wherein the compound is guanosine 5'-monophosphate and the hydrolase is a nucleosidase.

6. A process for producing 2'-deoxyguanosine as described in claim 5, wherein the nucleoside deoxyribosyl transferase is nucleoside deoxyribosyl transferase II; the nucleosidase is inosinate nucleosidase; and the 2'-deoxynucleoside is 2'-deoxypyrimidine nucleoside.

7. A process for producing 2'-deoxyguanosine as described in claim 5 or 6, wherein the 2'-deoxynucleoside is thymidine.

8. A process for producing 2'-deoxyguanosine as described in claim 1, wherein the compound is a 2-amino-6-substituted purine and the hydrolase is deaminase.

9. A process for producing 2'-deoxyguanosine as described in claim 8, wherein the nucleoside deoxyribosyl transferase is nucleoside deoxyribosyl transferase II; the deaminase is adenosine deaminase; and the 2'-deoxynucleoside is 2'-deoxypyrimidine nucleoside.

10. A process for producing 2'-deoxyguanosine as described in claim 8 or 9, wherein the 2-amino-6-substituted purine has a substituent which is hydrolizable.

11. A process for producing 2'-deoxyguanosine as described in claim 8 or 9, wherein the 2-amino-6-substituted purine is a 2-amino-6-halogenopurine.

12. A process for producing 2'-deoxyguanosine as described in claim 11, wherein the 2-amino-6-halogenopurine is 2-amino-6-chloropurine.

13. A process for producing 2'-deoxyguanosine as described in claim 8 or 9, wherein the 2-amino-6-substituted purine is 2,6-diaminopurine.

14. A process for producing 2'-deoxyguanosine as described in claim 8 or 9, wherein the 2'-deoxynucleoside is thymidine.
